# EUROPEAN PATENT SPECIFICATION

(11) **EP 2 679 243 B1**
(45) Date of publication and mention of the grant of the patent: **24.05.2017**
(21) Application number: 13425091.9
(22) Date of filing: 26.06.2013
(51) Int. Cl.: A61K 47/02, A61K 9/48, A61K 9/51, A61K 31/05

(54) **Co-precipitate of one or more stilbene polyphenols in lamellar anionic solids, it's applications and related preparation method**
Kopräzipitat von einem oder mehreren Polyphenolen in lamellenartigen anionischen Feststoffen, dessen Anwendungen und zugehöriges Herstellungsverfahren
Co-précipité d'un ou de plusieurs polyphénols de stilbène dans des solides anioniques lamellaires, les applications et le procédé de préparation

(30) Priority: 27.06.2012 IT PG20120030
(43) Date of publication of application: 01.01.2014
(73) Proprietor: Fioretti, Bernard, 06038 Spello (PG) (IT)
(72) Inventor: Fioretti, Bernard, 06038 Spello (PG) (IT); Spogli, Roberto, 06100 Perugia (PG) (IT); Sisani, Michele, 06100 Perugia (PG) (IT); Perioli, Luana, 06012 Cittá DI Castello (PG) (IT); Latterini, Loredana, 06053 Deruta (PG) (IT)
(74) Representative: Mauri, Elisabetta Maria Ester

(56) References cited:
- WO-A2-2010/016034
- US-A1- 2010 048 731
- US-A1- 2011 142 899
- EL-TONI A M ET AL: "Silica coating and photochemical properties of layered double hydroxide/4,4<'>-diaminostilbene-2,2<'>-di sulfonic acid nanocomposite", JOURNAL OF COLLOID AND INTERFACE SCIENCE, ACADEMIC PRESS, NEW YORK, NY, US, vol. 293, no. 2, 15 January 2006 (2006-01-15), pages 449-454, XP024909089, ISSN: 0021-9797, DOI: 10.1016/J.JCIS.2005.06.057 [retrieved on 2006-01-15]
- BATEMAN E D ET AL: "Efficacy and safety of roflumilast in the treatment of asthma", ANNALS OF ALLERGY, ASTHMA & IMMUNOLOGY, ARLINGTON HEIGHTS, IL, US, vol. 96, no. 5, 1 May 2006 (2006-05-01), pages 679-686, XP026959474, ISSN: 1081-1206 [retrieved on 2006-05-01]
- BAEUMER WOLFGANG ET AL: "Highly selective phosphodiesterase 4 inhibitors for the treatment of allergic skin diseases and psoriasis", INFLAMMATION & ALLERGY DRUG TARGETS, BENTHAM SCIENCE PUBLISHERS, NL, vol. 6, no. 1, 1 March 2007 (2007-03-01), pages 17-26, XP009147906, ISSN: 1871-5281, DOI: 10.2174/187152807780077318
- DINTER H ET AL: "Phosphodiesterase type IV inhibitors in the treatment of multiple sclerosis", JOURNAL OF MOLECULAR MEDICINE, SPRINGER VERLAG, DE, vol. 75, no. 2, 1 February 1997 (1997-02-01), pages 95-102, XP002113590, ISSN: 0946-2716, DOI: 10.1007/S001090050094
- D. C Grootendorst ET AL: "Reduction in sputum neutrophil and eosinophil numbers by the PDE4 inhibitor roflumilast in patients with COPD", Thorax, vol. 62, no. 12, 1 December 2007 (2007-12-01), pages 1081-1087, XP055319359, GB ISSN: 0040-6376, DOI: 10.1136/thx.2006.075937
- Thérèse Keravis ET AL: "Disease Progression in MRL/lpr Lupus-Prone Mice Is Reduced by NCS 613, a Specific Cyclic Nucleotide Phosphodiesterase Type 4 (PDE4) Inhibitor", PLoS ONE, vol. 7, no. 1, 11 January 2012 (2012-01-11), page e28899, XP055319360, DOI: 10.1371/journal.pone.0028899
- Takeya Kitta: "Type 4 phosphodiesterase inhibitor suppresses experimental bladder inflammation", BJUI, vol. 102, no. 10, 11 April 2008 (2008-04-11), pages 1472-1476, XP055319363,
- MCCANN FIONA E ET AL: "Apremilast, a novel PDE4 inhibitor, inhibits spontaneous production of tumour necrosis factor-alpha from human rheumatoid synovial cells and ameliorates experimental arthritis", ARTHRITIS RESEARCH AND THERAPY, BIOMED CENTRAL, LONDON, GB, vol. 12, no. 3, 2 June 2010 (2010-06-02), page R107, XP021085235, ISSN: 1478-6354, DOI: 10.1186/AR3041
- SUNG-JUN PARK ET AL: "Resveratrol Ameliorates Aging-Related Metabolic Phenotypes by Inhibiting cAMP Phosphodiesterases", CELL, CELL PRESS, US, vol. 148, no. 3, 5 January 2012 (2012-01-05), pages 421-433, XP028397795, ISSN: 0092-8674, DOI: 10.1016/J.CELL.2012.01.017 [retrieved on 2012-01-11]
- Pooneh Salari ET AL: "Phosphodiesterase inhibitors in inflammatory bowel disease", Expert Opinion on Investigational Drugs, vol. 21, no. 3, 6 February 2012 (2012-02-06), pages 261-264, XP055319366, UK ISSN: 1354-3784, DOI: 10.1517/13543784.2012.658915
- ULIBARRI M A ET AL: "HYDROTALCITE-LIKE COMPOUNDS AS POTENTIAL SORBENTS OF PHENOLS FROM WATER", APPLIED CLAY SCIENCE, ELSEVIER, AMSTERDAM, NL, vol. 10, no. 1/02, 1 August 1995 (1995-08-01), pages 131-145, XP002942207, ISSN: 0169-1317, DOI: 10.1016/0169-1317(95)00020-5
- José Manuel Lopez-Nicolás ET AL: "Aggregation State and p K a Values of ( E )-Resveratrol As Determined by Fluorescence Spectroscopy and UV-Visible Absorption", Journal of Agricultural and Food Chemistry, vol. 56, no. 17, 10 September 2008 (2008-09-10), pages 7600-7605, XP055319376, US ISSN: 0021-8561, DOI: 10.1021/jf800843e
- U.S. Environmental Protection Agency: "SCREENING-LEVEL HAZARD CHARACTERIZATION Stilbene Fluorescent Brightener Intermediates", Hazard Characterization Document, 1 March 2012 (2012-03-01), XP055319380, Retrieved from the Internet: URL:www.epa.org [retrieved on 2016-11-15]

## Description

Co-precipitate of one or more stilbene polyphenols in lamellar anionic solids, its applications and related preparation method.

### 1. APPLICATION FIELD.

In the present invention a new product is described (below, referred to as "product") made of compounds as defined in claim 1, which presents an increased dissolution rate in aqueous environment at gastric acid pH, an increased bioavailability, a greater photostability and fluency compared to stilbene polyphenols (below, referred to as "starting active principles").

The process to obtain this product is based on the development, in hydro-alcoholic environment, of a co-precipitate as defined in claim 1. The increased dissolution rate of the stilbene polyphenols co-precipitate, and therefore the better biovailability compared to the starting one, makes this product suitable for the preparation of formulations with improved activity and it allows its use for different applications. This invention allows formulations and applications to the pharmaceutical, nutraceutical alimentary, cosmeceutical and dermatological field and to medical devices. Considering the current knowledges on the activity of stilbene polyphenols, in particular of resveratrol, the improved pharmacokynetics properties are the anti-oxidant, anti-inflammatory, antiviral, antidiabetic, cardioprotective, neuroprotective, chemoprotective, antibiotic, antiischemica, anti-obesity, anti-hypertensive, anti-cancer, anti-tumor, anti-angiogenic ones. Also, it stands out for its greater ability to reduce melanoma and colon rectal cancer incidence, its greater efficacy in the regularization of glucose, triglycerides and inflammation markers levels in obese subjects, its ability to inhibit phosphodiesterase activity, particularly type 4, and therefore its use in treatment of chronic obstructive pulmonary diseases. This product, showing a greater photostability compared to the starting active principle, is also particularly suitable to industrial production and storage (increased *shelf life*). The previous observations can be extended to the whole class of stilbene polyphenols and their less soluble in water derivatives.

### 2. STATE OF THE ART

From a structural point of view, polyphenols are a heterogeneous group of compounds produced by plant secondary metabolism and characterized by the presence of phenolic groups. From a pharmacological and healthy point of view, polyphenols' activity can be summarized with the following actions: antioxidant, anticancer, antiteratogenic, anti-inflammatory, antibacterial and antiviral (Visioli et al., 2011, Polyphenols and human health: a prospectus. Crit Rev Food Sci Nutr.;5:524-46). A polyphenols' group which has aroused great interest for his healthy and pharmacological properties is represented by stilbene polyphenols (Roupe et al., 2006, Pharmacometrics of stilbenes: seguing towards the clinic. Curr Clin Pharmacol.;1:81-101). Stilbene polyphenols are compounds characterized by the following general formula (I): 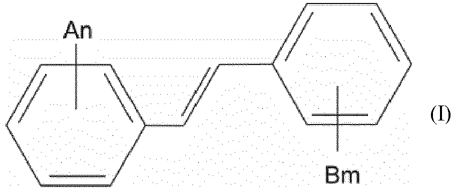 and relative multimers in which A and B are equal or different and each may represent an halogen atom, an amino group, an amido group, a sulfonic acid group, a phosphate group, a carboxylic group, -OR1 (where R1 may represent an hydrogen or an alchilic group from C1 to C5), a sugar residual, -OCOR2 (where R2 represents an hydroxil group or an alchilic group from C1 to C5); n and m are equal or different and each is an integer from 1 to 5.

Resveratrol (*Chemical Abstracts Service Registry Number* CAS 501-36-0) is a stilbenic structured polyphenol which can be found mainly in peel grapes and red wine, as well as in some plant species, for example Polygonum Cuspidatum, whose drug is represented by root. Resveratrol exists in two structural isomers (cis- (Z) e trans- (E)) and shows a characteristic instability at alkaline pH, while at pH values between 1 and 7 trans-resveratrol solutions are stable only when protected from light. Furthermore resveratrol is an extremely photosensitive compound. For example, trans-resveratrol is almost completely converted in cis-resveratrol (about 90%) as a result of irradiation for 120 minutes using wavelenght light of 366 nm (Trela and Waterhouse, J. Agric. Food Chem. 1996, 44, 1253-1257).
Compounds based on resveratrol, particularly in their trans form (3, 5, 4'-triidrossistilbene):
a) present antioxidant activity, anti-inflammatory, antiviral, cardioprotective, neuroprotective, chemoprotective, anticancer, antiangiogenic,
b) protect against infection and ischemia,
c) reduce obesity and prevent from ageing.
Animal researches demonstrated that resveratrol reduces melanoma and colon rectal cancer incidence. Furthermore reduces hypertension, heart attack incidence, glucose and triglyceride hematic level. In healthy obese human subjects, trans-resveratrol treatment reduces glucose, triglyceride and inflammation marker levels with an effect similar to that induced by caloric restriction.
(J M. Smoligal et al., 2011, Resveratrol and health - A comprehensive review of human clinical trials. Molecular Nutrition & Food Research -Special Issue: Resveratrol - 55:1129-1141*.*Vang et al., 2011, What is new for an old molecule? Systematic review and recommendations on the use of resveratrol. PLoS One. 2011;6(6*).*Timmers et al., 2011, Calorie restriction-like effects of 30 days of resveratrol supplementation on energy metabolism and metabolic profile in obese humans. Cell Metab. 14: 612-22*).*
Recently, it has been shown that resveratrol is an important inhibitor of phosphodiesterase IV (Park et al., 2012. Resveratrol ameliorates aging-related metabolic phenotypes by inhibiting cAMP phosphodiesterases. Cell. 2012 Feb 3;148(3):421-33.) demonstrating a therapeutic potential against pathologies such as chronic obstructive pulmonary disease (Chong et al., 2011. Phosphodiesterase 4 inhibitors for chronic obstructive pulmonary disease. Cochrane Database Syst Rev. 2011 May 11; (5): CD002309*.).*

Stilbene polyphenols and their derivatives, though being active principles of high pharmacological and healthy interest, thanks to their remarkable properties and applications, show limits in their use related to their reduced bioavailability. That is because of their low water solubility due to their lypophilicity and the absence of easily ionizable groups. For example, among stilbene polyphenols, resveratrol shows a reduced and variable bioavailability caused by his low solubility(Walle et al., 2004, High absorption but very low bioavailability of oral resveratrol in humans. Drug Metab Dispos. 32:1377-82*. -* Boocock et al., 2007, Phase I dose escalation pharmacokinetic study in healthy volunteers of resveratrol, a potential cancer chemopreveutive agent. Cancer Epidemiol Biomarkers Prev.16:1246-52*).*

The reduced bioavailability of resveratrol depends on its own chemical and physical features, poorly soluble in biological fluids with resulting pharmacokinetics problems, particularly during absorption of resveratrol at the gastro-intestinal level.
Therefore low bioavailability limits the efficacy of the active principle (S. Da et al., 2008, The impact of aqueous solubility and dose on the pharmacokynetic profiles od resveratrol. Pharmaceutical Research, 25: 2595-2600). Resveratrol is, infact, a poorly soluble in water compound (about 30mg/l), but very soluble in ethanol, and presents a high membrane permeability. According to the biopharmaceutical classification system (BCS), it is placed in class 2, which includes compounds that are poorly soluble and highly permeable (R. Löbenberg and Amidon 2000, Modern bioavailability, bioequivalence and biopharmaceutics classification system. New scientific approaches to international regulatory standards. European Journal of Pharmaceutics and Biopharmaceutics, 50, 3-12 *-* Amidon et al., 1995, A theoretical basis for a Biopharmaceutic Drug Classification: the correlation of in vitro drug product dissolution and in vivo bioavailability. Pharmaceutical Research, 12:413-420, adopted by the Food and Drug administration (FDA) to define dissolution assays of pharmaceutical solid forms for immediate release). Molecules belonging to this class show a high absorption number and a low dissolution number. This means that the step that limits the absorption rate of the active principle is represented by its dissolution rate in vivo. In this case, the absorption rate of the active principle depends strongly on the features of the formulation, on biological parameters like emptying gastric time, on interactions with food, on pathological and physiological conditions of the subject who ingests it, etc and limits healthy and pharmacological properties.

Following oral administration, resveratrol is observed in human plasma after thirty minutes, indicating that its absorption site corresponds also to the gastric absorption site. Certainly, absorption is present at intestinal level, where it has been supposed the presence of an active transport for resveratrol. Bioavailability of resveratrol is extremely variable. For example, a male subject's plasma concentration recorded following the administration of a single dose of resveratol of 25 mg corresponds to 10 ng/ml, but if the dose is increased by 20 times (500 mg daily) the concentration of plasma only increases by 7 times (72, 6 ng/ml). Other studies in which was used a pharmacological model based on daily administration by 150 mg for a prolonged period of time, observed additional disagreements as far as resveratrol's bioavailability is concerned: in one of these studies, plasma concentration, was equal to 231 ng/ml while in another studies was much lower (63.8 ng/ml)*(*Goldberg et al., 2003, . Absorption of three wine-related polyphenols in three different matrices by healthy subjects. Clin Biochem. 36:79-87. Boocock et al., 2007, Phase I dose escalation pharmacokinetic study in healthy volunteers of resveratrol, a potential cancer chemopreventive agent. Cancer Epidemiol Biomarkers Prev. 16:1246-52. Timmers et al., 2011, Calorie restriction-like effects of 30 days of resveratrol supplementation on energy metabolism and metabolic profile in obese humans. Cell Metab.14:612-22*.* Almeida et al., 2009, Pharmacokinetic and safety profile of trans-resveratrol in a rising multiple-dosestudy in healthy volunteers. Mol Nutr Food Res. 53 Suppl I:S7-15*).*

In order to solve the problem of resveratrol's bioavailability many strategies have been suggested (Amri A, Chaumeil JC, Sfar S, Charrueau C. Administration of resveratrol: What formulations solutions to bioavailability limitations? J Control Release. 2012 Mar 10;158(2):182-93*;* WO02/07591A2*).*

Particularly, the patent WO02/07591A2 describes the formation of a complex made of resveratrol and some phospholipids (the "complex") that increases lypophilicity and consequently favours gastro-intestinal absoprtion of the active principle (p. 2, line 15). In agreement with this complex's properties, the apparent permeability coefficient, measured using "Ussing Chamber's" method, is increased by about 8 times (p. 5, tab. 2, esempio 1). The Complex's formation is proved by spectroscopic studies (IR and H-NMR) that show the presence of intermolecular interactions caused by interactions between oxidrilic groups of resveratrol and phospholipids (p. 6, line 30).

This effects aren't ascribed to the possibility to form supramolecular phospholipidic structures as micelles, liposomes, unilamellar or polylamellar etc., but they could be compatible with a reverse micelle structure, considering the apolar organic solvent used (chloride acetylene, acetone, dioxane) and the absence of interactions with lypofilic chains of phospholipides, demonstrated by using spectrospic assays.

The complex identified as patent WO02/07591A2 is waxy and unlikely to be administered whereas, this invention, proposes a product in the form of powder, which presents a great flow aptitude and can be administered as it is or using traditional formulations as pills, capsules etc.

Another intrinsic limit of patent WO02/07591A2 lies in increasing the bioavailability by increasing the apparent permeability, while, as it happens with class 2 molecule of BCS system, the limiting step of stilbene polyphenols, particularly resveratrol and its derivatives, is represented by its dissolution rate. Therefore, the formation of lipofilic complexes or basic oil solution (for example soft capsules) isn't likely to solve solubility problems (as the present invention does). It only increases the crossing ability of biological membranes, in other words, the permeability.

Finally, in a specific way, phospholipides act as "permeability improving agents" producing, according to some studies, (Ilbäck et al., 2004. Do surface-active lipids in food increase the intestinal permeability to toxic substances and allergenic agents? Med Hypotheses, 63(4):724-30), an increase in frequency of pathologies such as cancer, cardiovascular diseases or diabetes, improving permeability in favor of toxic or allergenic substances, riducing healthy effects of stilbene polyphenols as resveratrol. The present invention allows, instead, to achieve a greater bioavailability through an important increase of the dissolution rate of stilbene polyphenols, respecting the permeability of biological membranes and their role as chemical and physical barrier from toxic substances and allergenic agents.

Patent WO2011/104667 describes Salt preparations of basic aminoacids and polyphenols as resveratrol. Particularly resveratrol-L-arginine salt presents an increased maximum plasma peak compared to that of resveratrol. Furthermore, resveratrol-L-arginine salt presents a better apparent solubility, spectroscopically measured, than resveratrol as it is.

Anionic hydrotalcytes and double hydroxide salts, which are lamellar anionic solids, caught the attention thanks to their various applications to the pharmaceutical, health, food and cosmeceutical field.

Hydrotalcites, also known as anionic clays or double lamellar hydroxides, are inorganic lamellar solids that do not contain organic carbon and thanks to their chemical structure, they represent a solid that is spatially organized as a lamellar sequence (Cap. 1, Vol. VII di Comprehensive Supramolecular Chemistry, Pergamon Press, Oxford, 1996). By using chemical methods it is possible to intercalate organic and inorganic anions whithin the interlamellar region of hydrotalcites. Then, molecules interposed in hydrotalcites are protected by oxidation reactions and photodegradation. Moreover, molecules interposed in hydrotalcites have been used as modified release systems and *drug carrier* (Carretero M. I., Lagaly G. (Eds.): Clays and Health Clays in Pharmacy, Cosmetics, Pelotherapy, and Environmental Projection. Appl. Clay Sci., 36, 2007*.;* Choy J. H., Oh J. M., Park D. H. Biomedical Applications of Layered Double Hydroxides in Low-Dimensional Solids, D.W. Bruce, D. O'Hare, R. I. Walton (Eds), chapter 3, 2010).

Two important properties of interposing compounds are the acidic feature and the water solubility. Unfortunately, compounds characterized by low water solubility and/or low acidity (as polyphenols, particularly resveratrol), are difficult to deprotonate, therefore the intercalation and the achievement of those properties previously described are very complicated.

A hydrotalcites structure can be given to brucite or Mg(OH)₂ which, after isomorphic substitution of Mg (M(II)) with another trivalent metal as (M(III)), acquire an excess of positive charge balanced by anions localized in interlayer region (for example Cl⁻ o NO₃⁻). The general formula of syntetic hydrotalcites or double lamellar hydroxides can be written as [M(II)₁₋ₓM(III)ₓ(OH)₂]^{x+}[Aⁿ⁻_{x/n}]^{x-} × mS where M(II) is a valency metal (II) preferably chosen amongst Mg, Zn, Co, Ni, Mn, Cu; M(III) is a valency metal preferably chosen amongst Al, Cr, Fe, V, Co; Aⁿ⁻ is an anion characterized by negative charge equal to n, that balances positive charge of lamelles: m is the number of solvent molecules (S), co-intercalated, by formula unit of the compound.

Hydroxide double salts (HDSs) are lamellar anionic solids formed by a unique cationic kind characterized by the same valency, usually divalent as Mg, Cu, Ni e Zn, where the anionic interlamellar exchange site is formed by differences in structure coordination (tetrahedric or octahedric) of divalent cation (Biswick et al., 2006, Journal of Solid state Chemistry 179: 49-55).

Lamellar anionic solids, from a structural point of view, are anisotropic systems and, when analyzed using X rays diffraction they show reflections related to a crystalline structure, from wich it is possible to acquire informations on interlayer distance. For example, hydrotalcite MgAl- NO₃ presents a typical reflection on plans (003) corresponding to an interlayer distance of 8.9 Å, while after the anion exchange of NO₃ with furosemide the hydrotalcite intercalated resultant (MgAl-furosemide) presents a basal reflection corresponding to an increased interlamellar distance equal to 22.2 Å (Perioli et al., 2011, Applied Clay Science, 53, 696-703).

In some cases, the interaction between hydrotalcites and organic host molecule could lead to a distortion of the general crystal structure, with the formation of "amorphous" or poorly organized structures, different by an "intercalated" hydrotalcite. [WO2013/060738A1, in order to manufacture products to be used in packaging to increase the shelf life of food, describes a process for preparation of a polymer composed by hydrotalcites intercalated with active molecules prepared using the co-precipitation method in a single step reaction. Hydrotalcite co-precipitate and the active principle (among which it is cited, though not been actually used, also resveratrol, whose selection as a molecule is limited to one application as a antioxidant) are wasted in a polymeric medium through traditional incorporation methods. The single step co-precipitation process is completely carried out in an aqueous solution. Intercalation products obtained this way, show a spectrum of rays X diffraction characterized by wide and not very intense peaks as evidence of structural deficiencies or low crystallinity].

The general formula of a hydroxide double salts (HDSs) can be written as [M(II)₁₋ₓM(II)₁₊ₓ(OH)_{3(1-y)}]^{x+}[N-][Aⁿ⁻]_{(1+3y)/n} × mS where M(II) is a valency metal (II) preferably chosen among Mg, Zn, Co, Ni, Mn, Cu; Aⁿ⁻ is an anion characterized by a negative charge n, which balances positive charge of lamelles; m is the molecules number of solvent (S), co-intercalated, for formula unity of compound.

### 3. SUMMARY OF INVENTION

Many of the problems described in the state of the art, about biovailability of stilbene polyphenols, among which resveratrol, are solved in this invention. This is made possible by co-precipitating one or more stilbene polyphenols, at alkaline pH, together with the lamellar anionic solids described above (hydroxide double salts (HDSs) and hydrotalcites) in a hydro-alcoholic environment.

The product obtained presents low crystallinity (or interlayers defects), as a result of an analysis carried out using spectrometry X rays diffraction, don't showing intense and wide peaks. Therefore, stilbenic structured polyphenol presents an anionic form, as highlighted throughout IR spectrum (data not shown).

The subject product of this invention shows a faster dissolution rate than stilbene polyphenols, reflected in a greater bioavailability of the active principle.

Therefore, the product is in powder, characterized by high purity, flow aptitude and photochemical stability, which makes it a product extremely suitable to industrial processes, without having to deal with dosage problems(feature that makes this product different from, for example, those described in Patent WO02/07591A2).

The improved photostability of this product compared to stilbene polyphenols allows a greater ease when it comes to production, dosage and storage and as stated in the state of art, permitting different application to the pharmaceutical, nutraceutical, alimentary and cosmeceutical field, such as:
a) solids preparation for oral and buccal use: powders, particles, granulars, particle, capsules, pills, sugared almonds, chewing gum, boluses, cachets, tablets, lozenges, healing chocolate;
b) liquid/semisolids preparations for oral use: syrups, elixirs, pastries, juices, decoctions, suspensions, emulsions, mucilage, herbal teas, medical wines, apothems, potions;
c) preparations for cutaneous applications: creams, ointments, unguents, gels, emulgels, lotiones, liniments, medical soaps, medical and not medical shampoos, lapis and pencils, cataplasmata, poultices, patches, oils, oilies, tinctures, wines, medicated bandages;
d) auricolar and nasal preparations: mouthwashes, gargles, auricolar lavages, auricolar cones, nasal lavages, swabs, oils, oilies, emulsions, ointment, gels, emulgels, lipogels, tablets, capsules, films, medical poultrices;
e) ophthalmic preparations: eye drops, ophthalmic inserts, eyewashes;
f) preparations administered throughout natural lower cavities, anal and vaginal:
   suppositories, capsules, tablets, enemas, lavenders, enemas, irrigations, douches, foams, tampons, candles, vaginal suppositories, foams, gel, emulgel, creams, ointments, films, suspensions, packes, emplastra, rings, tampons, medicated tampons, medicated gauges:
g) spray preparations for local, oral, buccal and inhalatory use.

Considering the improved biopharmaceutical characteristics, the greater bioavailability, and also the greater workability of this product and the scalability of the production process, another important aspect is represented by the use of this product in the pharmaceutical, nutraceutical, cosmeceutical, alimentary and medical field.

A characteristic feature of this invention is represented by the preparation of a product which includes trans-resveratrol co-precipitated in lamellar anionic solids. Co-precipitation is obtained at alkaline pH in hydro-alcoholic medium after mixing of an hydoralcoholic solution containing trans-resveratrol and an hydro-alcoholic solution containing trivalent(M III) or bivalent (M II)metal salts or mixtures composed by trivalent and bivalent metal salts. The process through which this product is made, can be subdivided into different phases: I) preparation of a trans-resveratrol solution; II) preparation of a solution of trivalent or bivalent metal salts or a mixture of bivalent and trivalent metal salts; III) co-precipitation of trans-resveratrol in lamellar anionic solids at pH between 8-11 after mixing of the two different solutions described at points I and II; IV) separation of co-precipitate; V) co-precipitated washing; VI) solvents evaporation.

The increased dissolution rate of resveratrol produced by the present invention causes a better pharmacokinetic behaviour determining stable absorption and an increased bioavailability of resveratrol, allowing a major use of its healthy and pharmacological properties.

### 4.SHORT DESCRIPTION OF FIGURES

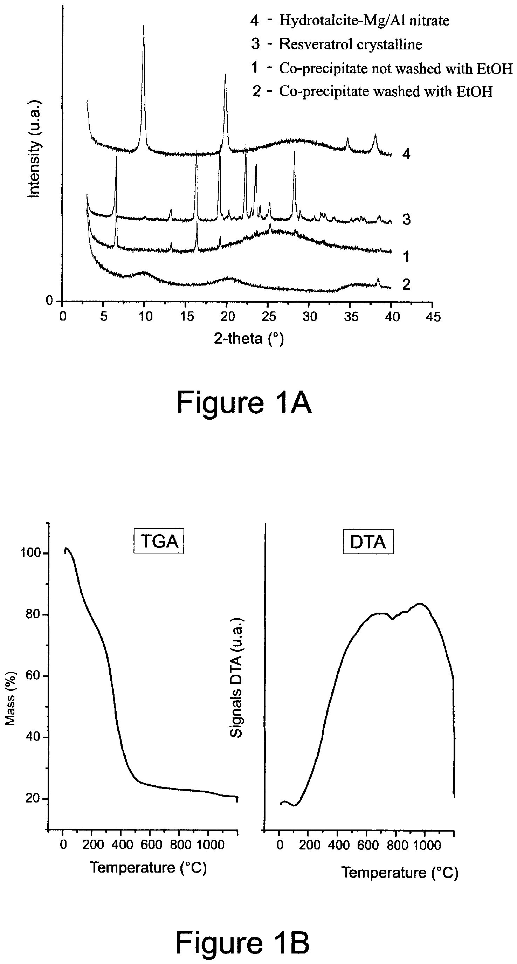

Figure 1. Characterization of the product obtained from the co-precipitation process according to the invention described in example 1. Figure 1A Spectrum of rays X diffraction on powders fro the co-precipitation product resulted from the combination of resveratrol with Mg and A1 nitrate salts. Spectra of rays X diffraction marked as number 1 and 2 show reflections of the co-precipitate obtained before (1) and after (2) ethanol washing, while diffractograms, marked as number 3 and 4 show reflections of hydrotalcite Mg/Al nitrate (4) and crystalline resveratrol (3, 100% purity). Figure 1B. The thermogravimetric analysis defines weight percentage of resveratrol load in co-precipitate. The curve in the graph on the left suggests a loss of weight during the thermogravimetric analysis and defines percentage weight of resveratrol load, while the curve in the graph on the right indicates DTA (Thermal Differential Analysis) signal where it is possible to observe a sequence of exothermic processes due to resveratrol degradation.

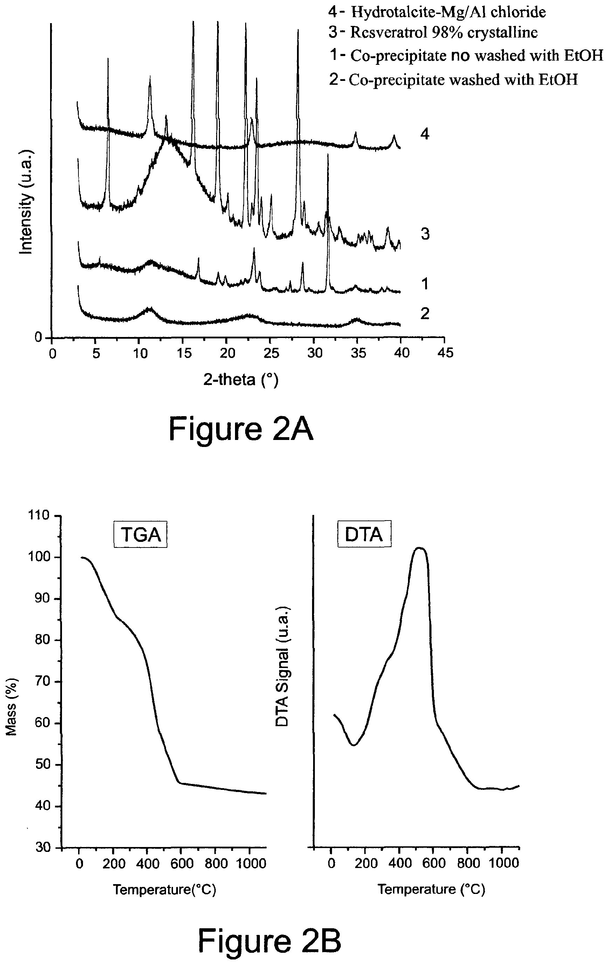

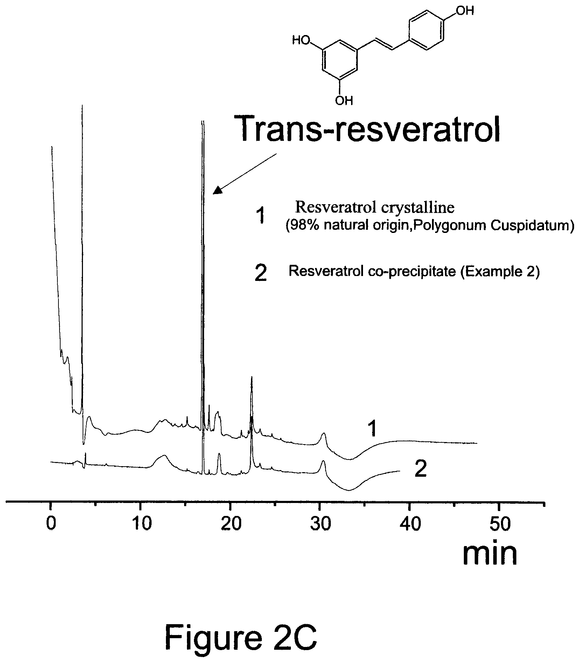

Figure 2. Characterization of the product obtained from the co-precipitation process according to the invention described in example 2. Figure 2A. Rays X diffraction spectrum on powders of co-precipitation product of resveratrol with Mg and A1 chloride salts. The spectra of rays X diffraction marked as 1 and 2 show reflections of the co-precipitate obtained according to the second example before (1) and after (2) ethanol washing, while spectra of rays X diffraction signed as 3 and 4 show reflections of Mg/Al chloride hydrotalcite and crystalline resveratrol (98% purity). Figure 2B. The thermogravimetric analysis to calculate weight percentage of resveratrol in co-precipitate. The curve in the graph on the left reveals a loss of weight during thermogravimetric analysis in order to determine the weight percentage of resveratrol, while the curve in the graph on the right shows DTA signal, wherein a sequence of exothermic processes due to resveratrol degradation is observed. Figure 2C. Cromatographic analysis of samples prepared from crystalline resveratrol (line 1) or from co-precipitate resveratrol described in example 2 (line 2). Samples are prepared in aqueous solution. To release resveratrol from co-precipitate in anionic hydrotalcites the aqueous solution is acidified at 1.2 pH. Column Luna Phenomenex RP-18, eluents A)-H₂O + TFA 0.1% e B- MeCN (Gradient: 5min: 5% B; 20 min: until 95% B; 5 min: up to 5% B, Temperature: 25°C, flow speed of eluent 1.0 mL/min, detector: DAD (190-400 nm))

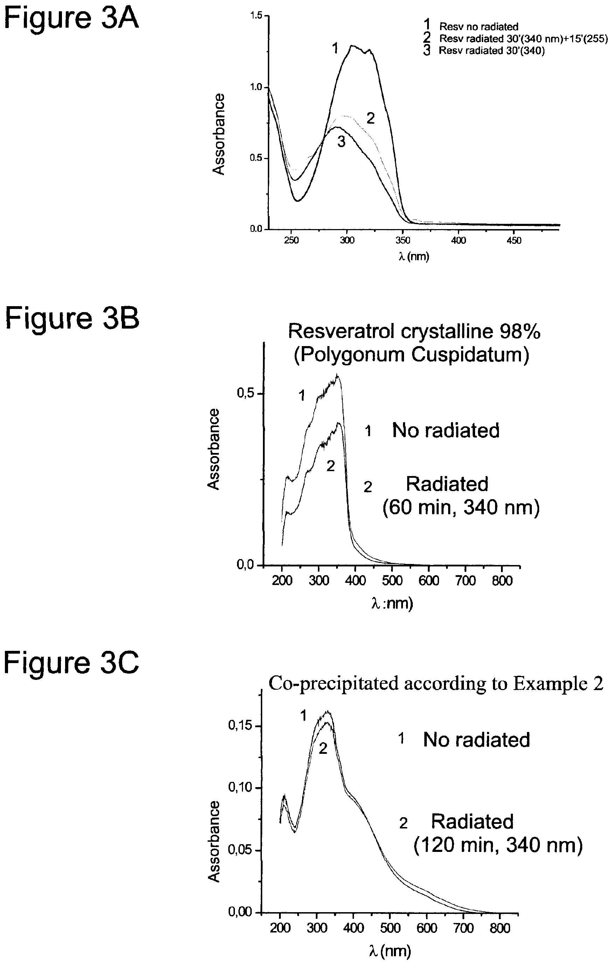

Figure 3. Resveratrol co-precipitates show a better photostability than crystalline resveratrol. A) Absorption spectrum of crystalline resveratrol. A) Absorption spectra of resveratrol in ethanol not irradiated (line 1), irradiated for 30 minutes (line 2) and after additional irradiation at 255 nm (line 3). B) Absorption spectra of crystalline resveratrol before irradiation (line 1) and after irradiation at 340 nm for 60 minutes (line 2). C) Absorption spectra of co-precipitated resveratrol according to example 2 before irradiation (line 1) and after irradiation at 340 nm for 120 minutes (line 2).

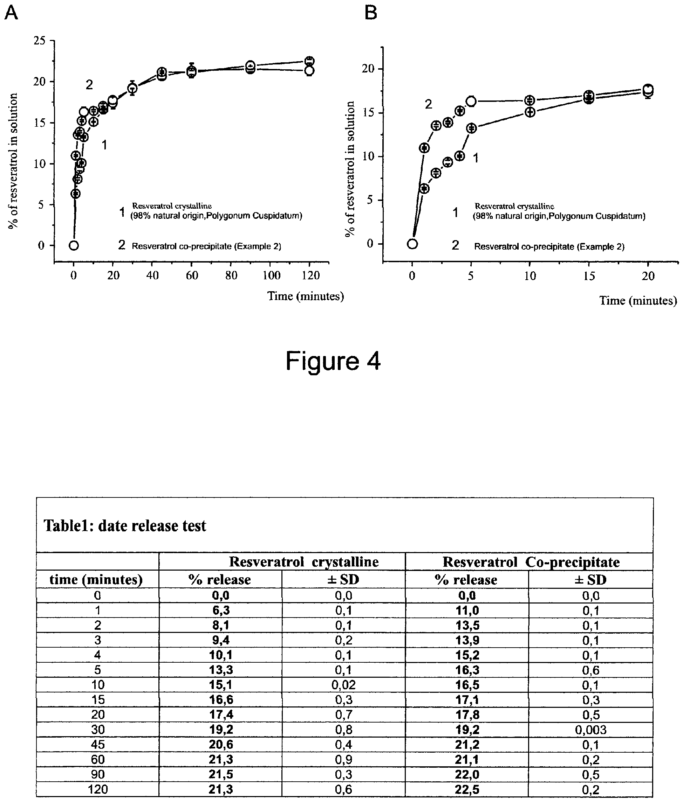

Figure 4. Co-precipitated resveratrol shows a better dissolution rate than crystalline resveratrol. A) The concentration of resveratrol in solution during the dissolution process is spectrophotometrically followed at 310 nm wavelenght and after performing a calibration line using gastric fluid simulated as white (r=0.9996). Experimental points marked as number 1 indicate resveratrol dissolution as it is (98% purity, Polygonum Cuspidatum) while those of co-precipitated resveratrol are marked as number 2. B) Detail of panel A. Concentration is expressed as resveratrol's percentage used for the study of release (132 mg). The dissolution rate is measured to acid gastric buffer (pH 1.2, XII italian FU) in non-sink condition at a temperature of 37°C and while being agitated (agitation 60 rotation/minute). Table 1 presents numeric values shown in panels A and B.

### 5. DETAILED DESCRIPTION OF THE INVENTION

The process through which stilbene polyphenols, among which resveratrol, and their co-precipitate derivatives, can be subdivided into different phases: I) Preparation of an hydro-alcoholic solution of one or more stilbene polyphenols; II) Preparation of an hydro-alcoholic solution composed by trivalent or divalent metal salts or a mixture of divalent and trivalent; III) Co-precipitation of one or more stilbene polyphenols in lamellar anionic solids at pH between 8-11 after mixing the solutions previously described at points I) and II); IV) co-precipitate separation; V) washing of co-precipitate; VI) solvents evaporation.

About the preparation of a solution composed by one or more stilbene polyphenols, described at phase I), this solution can be prepared dissolving one or more stilbene polyphenols in a hydro-alcoholic mixture at minimum concetration of 1 micromolar up to a maximum concentration equal to saturate concentration of stilbene polyphenols whitin the respective solution. The solution is kept stirred in a container under nitrogen flow and in the dark. PH of this solution can be included between 8 and 11, throughout addition of bases such as NaOH (to keep pH between 8 and 11).

Stilbene polyphenols are considered those molecules characterized by following general formula (I):

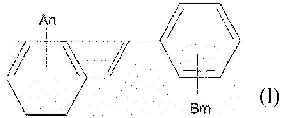

Examples of compounds of formula (I) are: trans-resveratrol (C14H12O3; CAS number 501-36-0); trans-3, 5-Dimethoxy-4'-hydroxy stilbene (C16H16O3; CAS n. 537-42-8); trans-3, 5, 4'-trimethoxy-stilbene (C17H18O3; CAS n. 22255-22-7); trans-3, 5-dihydroxy-4'-methoxy stilbene (C15H14O3; CAS n. 33626-08-3); trans-3, 5-diacetil-4'-hydroxy-stilbene (C18H16O5); trans-3, 5, 4'-triacetil-stilbene (C20H18O6); trans-3, 5-dihydroxy-4'-acetil-stilbene (C16H14O4); trans-piceide (C20H22O8; CAS n. 27208-80-6); trans-Pinosilvina (C14H12O2; CAS n. 102-61-4); cis-resveratrol (C14H12O3 CAS n. 61434-67-1); Raponticina (C21H24O9; CAS n. 155-58-8); trans Piceatannolo (C14H12O4; CAS n. 10083-24-6); Rapontigenina (C15H14O4; CAS n. 500-65-2); delta-Viniferina (C28H22O6; ChemSpider ID: 552752); epsilon-Viniferina (C28H22O6; CAS n. 62218-08-0); Astringina (C20H22O9; CAS n. 29884-49-9); Combretastatina A-1 (C18H20O6; CAS n. 109971-63-3); Hopeaphenol (C66H42O12; CAS n. 17912-85-5).

Stilbene polyphenols used in the preparation of solutions can be of synthetic or natural origin, characterized by different level of purity that range from 20 to 100% in weight, preferably from 50 to 100% and still more preferably from 98 to 100%. Stilbene polyphenols can be in cis or trans configuration, preferably in the trans one.

Regarding the preparation of salts solutions characterized by trivalent or divalent metal or mixture composed by both metal species, described in phase II) this solution can be achieved through the dissolution of the respective salts in a hydro-alcoholic mixture. Salts are binary compounds composed by divalent or trivalent metal and an anion preferably chosen among carbonates, sulfates, hydroxydes, phosphates, fluorides, chlorides, bromides, nitrates, iodides, perchlorates. Furthermore, divalent metal (II) is a valency metal preferably chosen among Mg, Zn, Co, Ni, Mn, Cu, while trivalent metal (III) is a valency metal preferably chosen among Al, Cr, Fe, V, Co. The solution can be prepared dissolving one salt, preferably composed by a divalent metal chosen among Mg, Zn, Co, Ni, Mn, Cu, or more salts (usually two) of divalent or trivalent metals. If the solution's case, it is composed by two salts, one of which derives from a divalent metal and the other from a trivalent metal, stechiometry is organized in order to have a percentage mole ratio of divalent metal compared to total moles of metals (total of divalent and trivalent moles) included in a range from 1 to 99%, or preferably from 30 to 70% and still more preferably from 60 to 65%.

An hydro-alcoholic solution is composed by water and one or more alcohols, preferably only one, characterized by a percentage composition in alcohol volume with respect to the total volume (water + alcohols) that ranges from 1 to 99%, preferably from 25 to 75 %, and still more preferably from 45 to 55%. Among alcohols, those who can be used are: methanol, ethanol, propanol, isopropanol, tert-butanol and n-butanol.

The aqueous solution presents a steady known residual and it is preferably distilled, deionized and deprived of carbonate ions.

About the co-precipitation procedure described at point III), this is obtained by mixing the solutions described at phases I) and II), under nitrogen flow, in a dark environment and adding, if necessary, a base chosen amongst NaOH, KOH, CsOH, Mg(OH)₂, to lead pH in a range comprised from 8 to 11, preferably from 9 to 10 and still more preferably to 9.5. The base can be added either in solid form or in aqueous solution, deprived of carbonate in a time interval comprised from 0.1 to 24 hours, or better from 1 to 1 hour and even better from 2 to 4 hours. The temperature can be kept at 15 to 90 degrees, preferably at 15 to 70 degrees and still more preferably at 20 to 50 degrees.
The separation process described at point IV) can either be performed through sedimentation, centrifugation, normal filtration or using filtration at reduced pressure. The washing process of co-precipitate described at point V) is obtained thanks to the re-suspension of the precipitate by using water, or alcohol, preferably ethanol, or hydro-alcoholic mixtures in order to remove stilbene polyphenols which re-precipitated during synthesis and which are not bound to a lamellar anionic solid. This suspension is filterd two or four times as described at point IV).
The process of the solvents evaporation described at point VI), consists in removing, to the compound obtained at point V), the solvent is drying by using an aired stove at a temperature comprised between 20 and 60 degrees, preferably between 30 and 50 degrees, and even more preferably at 40 degrees or through the removal of the solvent at a reduced pressure.

**Example 1:** preparation of resveratrol co-precipitate with hydrotalcites MgAl nitrate. Solution 1 is prepared, dissolving 641 mg of Mg(NO₃)₂ * 6H₂O, 401.4 mg of Al(NO₃)₃ * 9H₂O in 18 mL of deionized water. Solution 2 is prepared dissolving 300 mg of resveratrol (100 in purity of synthetic origin) in 18 ml of hydro-alcoholic solution. To the second solution, which is being kept in the dark, agitated and in nitrogen flow, are added 10 ml of NaOH 1M solution (up to pH between 10-11). Afterwards, to secnd solution, 16 ml of the first solution are added, together with 6 ml of NaOH 1 M to keep pH between 10-11 while these additions are being performed. Solid precipitate is left to be digested for 15 minutes and then it is separated through centrifugation. The solid recovered has to be washed two times using 30 ml of deionized water and, finally, using 20 ml of ethanol. The product is then dryed out at 50 °C.
Rays X diffraction analysis reveals the absence of resveratrol crystals after washing (comparison Figure 1A black and red, respectively before and after washing), while it is possible to observe the relative signals to reflections of MgAl hydrotalcites, in the form of nitrate. The width of signals is due to low crystallinity of hydrotalcitic matrix that can be referred to as an amorphous phase.
Tight signals of the unwashed co-precipitate are to be ascribed to crystalline resveratrol, being them superimposable based on the X rays diffraction analysis, performed on pure resveratrol (compare traces 1 and 3 in Figure 1A)
Thermogravimetric analysis shows two degradation steps (Figure 1B). From the curve of mass drop (red trace) one can observe a first step (up to 220 °C) due to the loss of crystallization water and at 1200 °C one can observe the loss of constitution water (dehydroxilation) together with the decomposition of interlayer anions (nitrate) and resveratrol. The presence of resveratrol is confirmed by DTA signal (black trace) in which a sequence of exothermic processes due to resveratrol degradation, can be observed.
By computations made taking into account the presence of hydrotalcite MgAl nitrate, that presents the following formula Mg₂Al(OH)₆(NO₃)* 2H₂O, percentage load of sample resveratrol equals 30 % of weight.

**Example 2:** preparation of resveratrol co-precipitate with hydrotalcite MgAl chloride. Solution 1is prepared by dissolving 258.3 mg of AlCl₃ * 6H₂O and 508.3 mg of MgCl₂ * 6H₂O in 18 mL of deionized water. Solution 2 is prepared by dissolving 300 mg of resveratrol (98 % of purity of natural origin, Polygonum Cuspidatum) in 18 ml of ethanol. A hydro-alcoholic solution is obtained by mixing the first and second solution and keeping it in a dark environment, agitated and in nitrogen flow. To this solution, under nitrogen flow, it has to be added 10 ml of NaOH solution 1 M up to a pH between 10-11. Solid precipitated is left to be digested for 15 minutes and then it is separated through centrifugation. The solid recovered, has to be washed 2 times using 30 ml of deionized water and finally using 20 ml of ethanol. Finally the solid recovered is dryed out at 50 °C.
Rays X diffraction analysis reveals the absence of resveratrol crystals after washing (comparison between Figure 2A black and red, respectively, before and after washing), while signals relative to reflections of hydrotalcite MgAl in chloride form keep being persistent. Width of signals is due to low crystallinity of hydrotalcitic matrix that can be ascribed to an amorphous phase. Tights signals present in unwashed co-precipitate can be ascribed to crystalline resveratrol being them superimposable through the rays X diffraction analysis, performed on natural resveratrol characterized by a 98% of purity (compare traces 1 and 3 in Figure 2A). Thermogravimetric analysis shows two degradation steps (Figure 2B). From the curve of mass drop (red trace) one can observe a first step (up to 220 °C) due to the loss of crystallization water and at 1200 °C one can observe the loss of constitution water (dehydroxilation) together with the decomposition of interlayer anions (chloride and resveratrol). The presence of resveratrol is confirmed by DTA signal (black trace) in which it is possible to observe a sequence of exhotermic processes due to resveratrol degradation. By the computations made considering the presence of hydrotalcite MgAl chloride, characterized by the following formulation Mg₂Al(OH)₆(Cl)* 2H₂O, the percentage load of sample resveratrol equals 23.5 in weight.
A chromatographic HPLC analysis (figure 2C) is performed on samples prepared with the co-precipitated resveratrol described in example 2 and starting resveratrol (98 % of purity of natural origin, Polygonum Cuspidatum). Chromatograms result similar in terms of quality: trans-resveratrol represents the principal chemical species together with other little impurities also present in starting resveratrol. All together these analysis indicate that resveratrol is not degrated during the preparation phase of the co-precipitate.
In aqueous solution trans-resveratrol shows photo-instability, as demonstrated by trans-cis photo-conversion after a 30 minute irradiation at 340 nm (Figure 3A black trace: not irradiated; green trace: irradiated).
In its crystalline form resveratrol results to be photo-degraded as proved by the absorption variation after a 60 minute irradiation at 340 nm (Figure 3B. red trace: not irradiated; black trace:irradiated). Co-precipitated resveratrol shows a greater photostability. Therefore, following irradiation (in the same experimental conditions in which resveratrol solution and crystalline resveratrol are irradiated), the spectrum recorded in reflected light results superimposable with that of a not irradiated sample (data not shown). Little changes are observed after a 120 minute irradiation at 340 nm (Figure 3C. black trace: not irradiated; red trace: irradiated).
Resveratrol co-precipitated presents an increased dissolution rate compared to crystalline resveratrol. Dissolution tests in fluids mimicking the gastric fluid (pH: 1-2, FU) at 37 °C, stirred and characterized by a blade rotation speed equal to 60 rotation/min in not sink conditions. Solubility of resveratrol in gastric fluid (pH 1.2, FU) determined both through indirect weighing and UV dosage, turns out to be 44 mg/L. For the dissolution test, a quantity equal to 132 mg of resveratrol equivalent to 134.7 mg of crystalline resveratrol (98 % of purity of natural origin, Polygonum Cuspidatum) and 561.6 mg of co-precipitated resveratrol obtained as in the example 2 (resveratrol load equal to 23.5 % in weight), was used. Resveratrol' dissolution has been followed by takings at a prearranged time and by determining its concentration, using a spectrophotometric assay at wavelenght of 310 nm. In figure 4 the dissolution rate of both crystalline and co-precipitated resveratrol are overlapping. Note that, co-precipitated resveratrol shows a better dissolution rate than starting resveratrol (compare numerical data in figure 4 table).

**Example 3** (comparison): a co-precipitate of resveratrol with hydrotalcites MgAl nitrate is prepared following the process described in patent WO 2013/060738 A1. A solution is obtained dissolving 641 mg of Mg(NO₃)₂ * 6H₂O and 401.4 mg of Al(NO₃)₃ * 9H₂O in 18 mL of deionized water. A second solution is prepared throughout dissolution of 1 mg of resveratrol in 18 ml of water (presence of resveratrol' crystals, index that it is impossible to prepare a solution characterized by greater concentration). Note that in the technique used in patent WO 2013/060738 A1, anionic solutions are prepared, but this technique cannot be performed because resveratrol has an high pKa (10 about) and therefore is not easily ionizable, and at alkalyne pH, that could contribute to getting a greater concentration of resveratrol in its ionic form. However, this would be unstable, as proved immediately by a change of color (index of photodegradation according to literature). The final solution is obtained in a single step, mixing the two different aqueous solutions previously described (metal and resveratrol) and adding a NaOH 1 M solution up to 7.5 pH in 4 hours. Finally, the mixture of reaction is filtered and washed 3 times using 30 ml of deionized water and, in the end, dried out at 50 °C.
Using TGA analysis and considering the presence of hydrotalcite MgAl nitrate, presenting the following chemical formula: Mg₂Al(OH)₆(NO₃) * 2H₂O, peecentage load of resveratrol is unlikely appreciable (less than 1% in weight).

**Example 4:** preparation of a stilbene polyphenols co-precipitate composed by trans resveratrol and trans-3, 5, 4'-triacetil-stilbene (C₂₀H₁₈O₆) with hydrotalcites of MgAl nitrate. Trans-3, 5, 4'-triacetil-stilbene (C₂₀H₁₈O₆) is prepared adding 1.5 ml (14.7 mmol) acetic anhydride to a solution of resveratrol 0.88 mmol in 0.5 ml of pyridine. The desired product is obtained after 40 minutes of stirr at room temperature precipitating it from the solution of reaction through cooling in water balanced with ice. Precipitated filtered has a mass of 230 mg and it is identified using spectroscopy ¹H-NMR. For the preparation of the co-precipitate taken into account, a first solution has to be prepared by dissolving 641 mg of Mg(NO₃)₂ * 6H₂O, 401.4 mg of Al(NO₃)₃ * 9H₂O in 18 mL of deionized water. Then, a second solution, by dissolving 150 mg of resveratrol and 230 mg of trans-3, 5, 4'-triacetil-stilbene in 18 mL of hydro-alcoholic solution. To the second solution, kept in a dark ennvironment, stirred and in nitrogen flow, are added 3 ml of a NaOH 1 M solution (up to pH between 10-11). Then, 16 ml of the first solution together with 3 ml of NaOH 1 M have to be added, in order to maintain a pH value between 10-11 during additions. Solid precipitated is left to be digested for 15 minutes, and then it is separated through centrifugation. Solid recovered is washed 2 times using 30 ml of deionized water and once using 20 ml of ethanol. This product is dried out at 50 °C. By TGA analysis and considering the presence of hydrotalcite MgAl nitrate, the following chemical formula: Mg₂Al(OH)₆(NO₃)* 2H₂O, percentage load of resveratrol and trans-3, 5, 4'-triacetil-stilbene in the sample is about 40% in weight.

**Example 5:** preparation of delta-Viniferina (C28H22O6; ChemSpider ID: 552752) co-precipitate with hydrotalcites MgAl chloride. Solution 1 has to be prepared, dissolving 258.3 mg of AlCl₃ * 6H₂O and 508.3 mg of MgCl₂ * 6H₂O in 18 mL of deionized water. Solution 2, has to be prepared, dissolving 600 mg of delta-Viniferina in 18 mL of ethanol. The hydro-alcoholic solution, obtained mixing the first and second solution together, is kept in a dark room, stirred and in presence of nitrogen flow. To this solution, 10 ml of NaOH 1 M solution up to pH between 10-11, under nitrogen flow, have to be added. Solid precipitated is left to be digested for 15 minutes, and then it has to be separated through centrifugation. The solid recovered is washed 2 times using 30 ml of deionized water and once using 20 ml of ethanol. Finally it has to be dried out at 50 °C. By TGA analysis and considering the presence of hydrotalcite MgAl chloride, characterized by the following chemical formula: Mg₂Al(OH)₆(Cl)* 2H₂O, the percentage load of delta-Viniferina in sample is about 35.5% in weight.

**Example 6:** preparation of Combretastatina A-1 (C18H20O6; CAS n. 109971-63-3) co-precipitate with hydrotalcites MgAl nitrate. The first solution has to be prepared by dissolving 641 mg of Mg(NO₃)₂ * 6H₂O, 401.4 mg of Al(NO₃)₃ * 9H₂O in 18 mL of deionized water. The second solution has to be prepared dissolving 300 mg of Combretastatina A-1 in 18 mL of hydro-alcoholic solution. To second solution, kept in a dark room, agitated and in presence of nitrogen flow, 3 ml of NaOH 1M solution (up to pH between 10-11)have to be added. Afterwards, 16 ml of first solution with 6 ml of NaOH 1 M to maintain pH value between 10-11 during the co-precipitation, have to be added. Solid precipitated is left to be digested for 15 minutes and then it is separated through centrifugation. The solid recovered is washed 2 times using 30 ml of deionized water and once using 20 ml of ethanol. Consequently the product is dried out at 50 °C. Using TGA analysis and considering the presence of hydrotalcite MgAl nitrate, characterized by the following chemical formula: Mg₂Al(OH)₆(NO₃)* 2H₂O, Combretastatina A-1 percentage load in the sample is about 32% in weight.

**Example 7:** hard capsule 25 mg for pharmaceutical and nutraceutical use.

Co-precipitate described in example 1 (loading 30%) 83, 3 mg
capsule blend qb

Capsule standard blend is composed by lactose 75 %, starch 15%, colloidal anhydrous silica 7%, and magnesium stearate 3%. This suitable blend is used in variable amount depending on the size of the chosen capsule, changeable from 00 to 5, and filling method (manual, semiautomatic, auomatic). The intoduction of the material, object of the patent, into the hard capsules is a simple (also suitable for galenic use) and cheap process and it is not noxious for the material (there are no operations such as the use of solvents, high temperatures etc.)

**Example 8:** 25 mg tablet, fast release formulation for pharmaceutical and nutraceutical use.

| | |
|---|---|
| Co-precipitate described in first example (e.g. loading 30%) | 83.3 mg |
| Starch | 6.7 mg |
| sodium starch glycolate (Explotab®) | 10.0 mg |

Tablet, presenting a total weight of 100 mg, is prepared by using a of 13 mm diameter punch for direct compression (time 1 min, compression force of 5x103 Kg) by using a manual hydraulic press (Perkin Elmer, England). Compression occurs at low times and controlled compression force without use of lubricants or glidants, due to the special properties of lamellar anionic solids. High auto-compaction property of the product could permit tablet preparation without the support of other "ingredients". In this way however tablet disgregation and dissolution time would be prolonged too much. For this reason, the use of a disintegrant product is advantageous. The chosen disintegrant Explotab® produces a macroscopic effect on tablet disgregation and it is not responsible for active ingredient solubility increase. Its amounts in the tablet is 10%.

**Example 9:** 25 mg tablet, fast release, super type, for pharmaceutical and nutraceutical use.

| | |
|---|---|
| Co-precipitate described in first example (eg. loading 30%) | 83,3 mg |
| Amido | 12,7 mg |
| L-HPC LH-21® | 4,0 mg |

The tablet, total weight 100 mg, is prepared using a 13 mm diameter punch by direct compression (time 1 min, compression force of 5x10³ Kg) by using a hydraulic manual press (Perkin Elmer, England). For compression modalities and the choice to use a disintegrant product, take into account the observations in example 8.
In this case a new disintegrant, owing to new generation of superdisintegrant compounds, is employed. It is the superdisintegrant L-HPC LH-21® (cellulose 2-hydroxy prophylether low substituted; its employement ia useful to disgregate the tablet in a very fast manner and, also in this case, its activity is not responsible for active ingredient solubility increase. This super disgregant swells when put in contact with water thanks to multilayer sheet formation and to development of channels able to facilitate the water penetration. L-HPC LH-21® is water insoluble but able to absorbs a sufficient water amount to expand highly its volume. This swelling action is responsible for very fast tablet disaggregation. Swelling volume depends on superdisintegrant particle size, on glucose and hydro propoxy groups presents on the polymer chain. Taking into consideration its features, it can be used in a lower percentage in comparison to classic disintegrant; specifically 4%.

**Example 10:** Hydrophilic monophase formulation - hydrogel 2% for dermatologic and cosmeceutical use.

| | |
|---|---|
| Co precipitate described in example 1 (eg. loading 30%) | 2,0 g |
| Propilene Glycol | 10,0 g |
| Carboxy Methyl Cellulose sodium salt (NaCMC) | 5,0 g |
| Distilled water | until 100,0 g |

This formulation is formed by a classic hydrophilic gel based on pH independent gelling agent (cellulose derivative). This formulation is very simple and the product, purpose of this patent, is perfectly enclosed in this kind of formulation and even improves it.
Furthermore, the material of this patent is easily water dispersible and the only expedient to be taken into account, in order to obtain a very good formulation, is the preparation of primary its mixing with gelling polymer in anhydrous form that must be then moistened by adding propylene glycol in order to avoid floating phenomena and to incorporate all the new gel. The base gel improvement due to the addition of material of this patent is represented by rheological property improvement. Furthermore, hydrophilic gels as such are generally rigid and stiff, they show a plastic behaviour (low flow). The addition of lamellar materials is able to reduce the fluid flow limit (yield stress) and generally can transform plastic materials into pseudo-plastic materials.

**Example 11:** Hydrophilic multiphase preparation - 2% active in O/W emulsion for cosmeceutic use.

| | |
|---|---|
| co-precipitate described in example 1 (eg. loading 30%) | 2,0 g |
| **Oil phase (O)** | |
| vaseline | 11,0 g |
| mineral oil (paraffine) | 10,0 g |
| cethostearilyc alchol | 7,2 g |
| cetomacrogol 1000 | 1,8 g |
| **Water phase (W)** | |
| Hydroxy Ethyl cellulose (HEC) | 2,0 g |
| Glycerol | 10,0 g |
| Distilled water | until 100,0 |

The nature of the product (its chemical-physical properties) allows its additions both into phase W and phase O. The easier preparation method is the primary mixture of co-precipitate product with HEC followed by glycerol moistening and final preparation of gel by adding water. The final gel is added to hot melted oil phase (generally in a steam bath). In this way the active ingredient will be in the external phase of the emulsion (W) and therefore it will be easily available (released).

**Example 12:** Antiseptic mucoadhesive vaginal formulation (multiphase)
- emulsion O/W 2% - medical device (borderline M. D.).
-

| | |
|---|---|
| Co-precipitate described in example 1 (eg .loading 30%) | 2,0 g |
| **oil phase (O)** | |
| vaseline | 15,0 g |
| mineral oil (paraffine) | 6,0 g |
| cethostearilyc alchol | 7,2 g |
| **water phase (W)** | |
| cetomacrogol 1000 | 1, 8 g |
| NaCMC 4% gel | until 100, 0 g |

As regard the preparation method all the given indications of example 11 must be taken into account. The present biphasic formulation must also be bioadhesive (mucoadhesive) with the aim to adhere to vaginal mucosa in order to fix the formulation to the specific site of action. This has the double purpose to prevent the loss of material (also in orthostatic position of user) and to prolong the time contact between the active ingredient and the site of action. The presence of active ingredient in the external phase (W) of the emulsion allows its fast release and rapid antiseptic effect. If the active ingredient was in the internal phase (O), it should first pass from O to W and then from W to vaginal mucosa (release phase). The presence of lamellar material in the external phase (W) doesn't change the mucoadhesivity of this formulation and increases its flow properties which are very important for this kind of vaginal dosage form, both in the case of direct application *in loco* (increased spreadability) and in the case of a vaginal applicator is used.
When cream administration is performed by a vaginal applicator, the formulation had to go first from cream tube to applicator (increased extraction, increased extrudibility) and then from applicator to vaginal mucosa (enhanced flow, spreadability and expandibility).

## Claims

1. Product composed by one or more stilbene polyphenols co-precipitate in lamellar anionic solids, wherein:
a) stilbene polyphenols
are selected from the group consisting of trans-resveratrol ; trans-3,5-Dimethoxy-4'-hydroxy stilbene ; trans-3,5,4'-trimethoxy-stilbene; trans-3, 5-dihydroxy-4'-methoxy stilbene ; trans-3,5-diacetil-4'-hydroxy-stilbene ; trans-3,5,4'-triacetil-stilbene ; trans-3,5-dihydroxy-4'-acetil-stilbene ; trans-piceide ; trans-Pinosilvina ; cis-resveratrol ; Raponticina ; trans Piceatannolo ; Rapontigenina ; delta-Viniferina ; epsilon-Viniferina ; Astringina; Combretastatina A-1 ;and Hopeaphenol ;
b) lamellar anionic solids means:
- hydrotalcites, **characterized by** the following formula:
[M(II)1-ₓM(III)ₓ(OH)₂]^{x+}[Aⁿ⁻_{x/n}]^{x-} × mS
where M(II) is a valency metal (II) preferably chosen amongst Mg, Zn, Co, Ni, Mn, Cu; M(III) is a valency metal (III) preferably chosen amongst Al, Cr, Fe, V, Co; Aⁿ⁻ is an anion **characterized by** negative charge equal to n, that balances positive charge of lamelles: m is the number of solvent molecules (S),generally water, co-intercalated, by formula unit of the compound;
- the hydroxide double salts (HDSs) **characterized by** the following formula:
[M(II)₁₋ₓM(II)₁₊ₓ(OH)_{3(1-y)}]^{x+}[Aⁿ⁻]_{(1+3y)/n} × mS
where M(II) is a valency metal (II) preferably chosen amongst Mg, Zn, Co, Ni, Mn, Cu; Aⁿ⁻ is is an anion **characterized by** a negative charge n, which balances positive charge of lamelles; m is the number of solvent molecules (S), co-intercalated, for formula unity of compound.

2. Method for preparating a product composed by one or more stilbene polyphenols as defined in claim 1 co-precipitate with lamellar anionic solids as defined in claim 1, **characterized by** the following phases:
preparation of a hydro-alcoholic solution of one or more stilbene polyphenols ;
II) preparation of a hydro-alcoholic solution of divalent or trivalent metal salts or a mixture composed by divalents and trivalents;
III) co-precipitation of one or more stilbene polyphenols in lamellar anionic solids at pH between 8-11 following the mixture of solutions described at point I) and II) and the addition of hydroxides;
IV) separation of co-precipitate;
V) washing of co-precipitate;
VI) solvents evaporation.

3. The product according to claim 1) or the method according to claim 2), wherein amongst stilbene polyphenols are included both those of synthetic and natural origin, **characterized by** different level of purity, between 20 and 100%.

4. The use of the product composed by stilbene polyphenols and their co-precipitate with lamellar anionic solids as defined in claim 1) and realized according to the process described in claim 2) in a medical device.

5. The use of the product composed by stilbene polyphenols and their co-precipitate with lamellar anionic solids as defined in claim 1) and realized according to the process described in claim 2) for food aims.

6. The use of the product composed by stilbene polyphenols and their co-precipitate with lamellar anionic solids as defined in claim 1) and realized according to the process described in claim 2) for nutraceutical aims.

7. The use of the product composed by stilbene polyphenols and their co-precipitate with lamellar anionic solids as defined in claim 1) and realized according to the process described in claim 2) for formulating pharmaceutical products.

8. The use of the product composed by stilbene polyphenols and their co-precipitate with lamellar anionic solids as defined in claim 1) and realized according to the process described in claim 2) for dermatological aims.

9. The use of the product composed by stilbene polyphenols and their co-precipitate with lamellar anionic solids as defined in claim 1) and realized according to the process described in claim 2) for cosmeceutical aims.

10. The use according to claims 4), 5), 6), 7), 8) and 9), of the product composed by stilbene polyphenols and their co-precipitate with lamellar anionic solids as defined in claim 1) and realized according to the process described in claim 2), singularly or in association with ingredients, in specific formulations for:
a) solid preparations for oral and buccal use: powders, granulates, particle, capsules, pills, tablets, coated tablets, chewing gums, bolus, cachets, losanges, medicated chocolates;
b) liquid/semisolid preparations for oral use: syrups, elixir, pastes, juices, decoctions, suspensions, emulsions, mucillagines, herb tea, medicated wines, apothems, potions;
c) preparations for skin application: creams, pastes, ointments, gels, emulgels, lotions, liniments, medicated soaps, shampoos and medicated shampoos, lapis or pencils, cataplasmata, emplastra, patches, oils, oleolites, oleose, tinctures, wines, medicated gauges;
d) preparations for auricolar, nasal and oro mucosal use: mouthwashes, gargles, auricolar washing, auricolar cone-shaped, nasal washing, tampons, oils, oleolites, emulsions, ointments, gels, emulgels, tablets, capsules, films, patches, emplastra;
e) ophthalmic preparations: collyria, ocular inserts and ocular whashing;
f) preparations to be administered on natural cavities (anal and vaginal routes): suppositories, capsules, tablets, enema, enteroenemas, enema, irrigations, douches, foams, tampons, candles, ovoles, foams, gels, pastes, emulgels, creams, ointments, films, suspensions, packes, emplastra, rings, medicated tampons, medicated gauges;
g) spray preparations for topical, inhalatory, oral, buccal uses.

11. The use according to claims 4), 5), 6), 7), 8), 9) and 10), of the product composed by stilbene polyphenols and their co-precipitate with lamellar anionic solids defined in claim 1) and realized according to the process described in claim 2) in formulations presented in claim 10) **characterized by** the following properties:
a) antioxidant, antiinflammatory, anti-viral, anti-diabetes, hearth-protection, neuronal protection, chemo-protection, antibiotic, anti-ischemic, anti-obesity, antihypertensive, anti-cancer, antitumor, anti-angiogenic and anti-ageing activities;
b) protective activities against infective agents, cardiovascular dysfunction and neoplastic disease such as melanoma and colon-rectal cancer;
c) regularizing activity in glucose and triglyceride blood levels and inflammatory markers in human obesity;

12. A resveratrol co-precipitate with lamellar anionic solids as defined in claim 1) and realized according to the process described in claim 2) in specific formulations as described in claim 10) for use according to claims 4), 5), 6), 7), 8), 9) and 10),
a) in a method of treating oxidation, inflammation, viral infections, diabetes, hearth diseases, neuronal diseases, chemicals adverse effects, bacterial infections, ischemia, obesity, hypertension, cancer, tumor, angiogenesis, age activities;
b) in a method of preventing infective diseases, cardiovascular dysfunction and neoplastic disease such as melanoma and colon-rectal cancer;
c) in a method of regularizing activity in glucose and triglyceride blood levels and inflammatory markers in human obesity.

13. The production and industrial use according to claims from 2) to 12), of the product composed by one or more stilbene polyphenols and their derivatives co-precipitate with lamellar anionic solids defined in claim 1) realized according to the process described in claim 2) tel quel and/or in formulations; particularly in those procedures where better photostability of product produces advantages in manipulation of stilbene polyphenols during production and storage of these last.

14. A product composed by resveratrol and lamellar anionic solids defined in claim 1) realized according to the process described in claim 2) for use according to claims 4), 5), 6), 7), 8), 9) and 10) in the treatment ofpathologies linked to phosphodiesterase IV inhibition,

15. A product composed by resveratrol and lamellar anionic solids defined in claim 1) realized according to process described in claim 2) for use according to claims 4), 5), 6), 7), 8), 9) and 10), in treatment of chronic obstructive pulmonar disease.

## Patentansprüche

1. Produkt, zusammengesetzt aus einem oder mehreren Stilbenpolyphenolen zusammen ausgefällt in lamellaren anionischen Feststoffen, wobei:
a) Stilbenpolyphenole ausgewählt sind aus der Gruppe bestehend aus trans-Resveratrol; trans-3,5-Dimethoxy-4'-hydroxystilben; trans-3,5,4'-Trimethoxystilben; trans-3,5-Dihydroxy-4'-methoxystilben; trans-3,5-Diacetyl-4'-hydroxystilben; trans-3,5,4'-Triacetylstilben; trans-3,5-Dihydroxy-4'-acetylstilben; trans-Piceid; trans-Pinosylvin; cis-Resveratrol; Rhaponticin; trans-Piceatannol; Rhapontigenin; delta-Viniferin; epsilon-Viniferin; Astringin; Combretastatin A-1 und Hopeaphenol;
b) lamellare anionische Feststoffe bedeuten:
- Hydrotalkite, **gekennzeichnet durch** die folgende Formel:
[M(II)₁₋ₓM(III)ₓ(OH)₂]^{x+}[Aⁿ⁻_{x/n}]^{x-} × mS,
wobei M(II) ein Valenzmetall (II) ist, das vorzugsweise aus Mg, Zn, Co, Ni, Mn, Cu ausgewählt ist; M(III) ein Valenzmetall (III) ist, das vorzugsweise aus Al, Cr, Fe, V, Co ausgewählt ist; Aⁿ⁻ ein Anion ist, das **durch** eine negative Ladung gleich n gekennzeichnet ist, die eine positive Ladung von Lamellen ausgleicht: m die Anzahl von Lösungsmittelmolekülen (S) ist, im allgemeinen Wasser, die **durch** die Formeleinheit der Verbindung co-interkaliert sind;
- die Hydroxid-Doppelsalze (HDSs), die **durch** die folgende Formel gekennzeichnet sind:
[M(II)₁₋ₓM(II)₁₊ₓ(OH)_{3(1-y)}]^{x+}[Aⁿ⁻]_{(1+3y)/n} × mS,
wobei M(II) ein Valenzmetall (II) ist, das vorzugsweise aus Mg, Zn, Co, Ni, Mn, Cu ausgewählt ist; Aⁿ⁻ ein Anion ist, das **durch** eine negative Ladung n gekennzeichnet ist, die die positive Ladung von Lamellen ausgleicht; m die Anzahl von Lösungsmittelmolekülen (S) ist, die für die Formeleinheit der Verbindung co-interkaliert sind.

2. Verfahren zur Herstellung eines Produkts, zusammengesetzt aus einem oder mehreren Stilbenpolyphenolen nach Anspruch 1 zusammen ausgefällt mit lamellaren anionischen Feststoffen nach Anspruch 1, **gekennzeichnet durch** die folgenden Phasen:
I) Herstellung einer hydroalkoholischen Lösung aus einem oder mehreren Stilbenpolyphenolen;
II) Herstellung einer hydroalkoholischen Lösung aus zweiwertigen oder dreiwertigen Metallsalzen oder einer Mischung, die aus Zweiwertigen und Dreiwertigen zusammengesetzt ist;
III) Mitfällung von einem oder mehreren Stilbenpolyphenolen in lamellaren anionischen Feststoffen bei einem pH-Wert zwischen 8-11 nach dem Mischen von Lösungen beschrieben in Punkt I) und II) und der Zugabe von Hydroxiden;
IV) Trennung der Mitfällung;
V) Waschen der Mitfällung;
VI) Lösungsmittel-Verdunstung.

3. Produkt nach Anspruch 1) oder Verfahren nach Anspruch 2), wobei unter den Stilbenpolyphenolen sowohl solche synthetischen als auch natürlichen Ursprungs enthalten sind, die durch einen unterschiedlichen Reinheitsgrad zwischen 20 und 100% gekennzeichnet sind.

4. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren in einer medizinischen Vorrichtung.

5. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren für Ernährungszwecke.

6. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren für nutrazeutische Zwecke.

7. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren zur Formulierung von pharmazeutischen Produkten.

8. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren für dermatologische Zwecke.

9. Verwendung des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren für kosmezeutische Zwecke.

10. Verwendung nach Ansprüchen 4), 5), 6), 7), 8) und 9) des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren, einzeln oder in Verbindung mit Inhaltsstoffen, in spezifischen Formulierungen für:
a) feste Zubereitungen zur oralen und bukkalen Verwendung: Pulver, Granulate, Partikel, Kapseln, Pillen, Tabletten, Dragees, Kaugummis, Bolus, Kapseln, Pastillen, medizinische Schokolade;
b) flüssige/halbfeste Zubereitungen zur oralen Verwendung: Sirupe, Elixiere, Pasten, Säfte, Aufgüsse, Suspensionen, Emulsionen, Schleimstoffe, Kräutertee, medizinische Weine, Apothema ("apothems"), Tränke;
c) Zubereitungen zur Hautanwendung: Cremes, Pasten, Salben, Gele, Emulgele, Lotionen, Einreibemittel, medizinische Seifen, Shampoos und medizinische Shampoos, Lapis oder Stifte ("lapis or pencils"), Wickel, Pflaster, Patches, Öle, Oleolite ("oleolites"), Oleose ("oleose"), Tinkturen, Weine, medizinische Lehren;
d) Zubereitungen für aurikolare, nasale und oro-mukosale Verwendung: Mundspülungen, Gurgelmittel, aurikulare Spülungen, aurikulare Kegelförmige, Nasenspülung, Tampons, Öle, Oleolite, Emulsionen, Salben, Gele, Emulgele, Tabletten, Kapseln, Filme, Patches, Pflaster;
e) ophthalmische Zubereitungen: Augenspülungen, Augeneinsätze und Augenspülung;
f) Zubereitungen, die auf natürlichen Kavitäten (Anal- und Vaginalrouten) angewendet werden sollen: Zäpfchen, Kapseln, Tabletten, Einlauf, Darm-Einläufe, Einlauf, Spülungen, medizinische Spülungen, Schäume, Tampons, Kerzen, Ovolen (ovoles), Schäume, Gele, Pasten, Emulgele, Cremes, Salben, Filme, Suspensionen, Packungen, Pflaster, Ringe, medizinische Tampons, medizinische Lehren;
g) Sprühzubereitungen für topische, inhalatorische, orale, bukkale Verwendungen.

11. Verwendung nach Ansprüchen 4), 5), 6), 7), 8), 9) und 10) des Produkts, zusammengesetzt aus Stilbenpolyphenolen und deren Mitfällung mit lamellaren anionischen Feststoffen nach Anspruch 1) und realisiert nach dem in Anspruch 2) beschriebenen Verfahren in Formulierungen nach Anspruch 10), **gekennzeichnet durch** die folgenden Eigenschaften:
a) antioxidative, entzündungshemmende, antivirale, Anti-Diabetes-, Herzschutz-, Neuronenschutz-, Chemoschutz-, antibiotische, anti-ischämische, anti-Adipositas-, antihypertone, anti-Krebs-, anti-Tumor-, anti-angiogene und Anti-Aging-Aktivitäten;
b) schützende Aktivitäten gegen infektiöse Wirkstoffe, kardiovaskuläre Dysfunktion und neoplastische Erkrankung wie zum Beispiel Melanom und Darmkrebs;
c) Regulierung der Aktivität in Glukose- und Triglycerid-Blutspiegeln und entzündlichen Markern bei menschlicher Adipositas.

12. Resveratrol-Mitfällung mit lamellaren anionischen Feststoffen nach Anspruch 1) und realisiert nach dem in Anspruch 2) beschriebenen Verfahren in spezifischen Formulierungen nach Anspruch 10), zur Verwendung nach Ansprüchen 4), 5), 6), 7), 8), 9) und 10),
a) in einem Verfahren zur Behandlung von Oxidation, Entzündung, viralen Infektionen, Diabetes, Herzerkrankungen, neuronalen Erkrankungen, chemischen Beeinträchtigungen, bakteriellen Infektionen, Ischämie, Adipositas, Hypertonie, Krebs, Tumor, Angiogenese, Alter-Aktivitäten;
b) in einem Verfahren zur Vermeidung von infektiösen Erkrankungen, cardiovaskulärer Dysfunktion und neoplastischer Erkrankung wie zum Beispiel Melanom und Darmkrebs;
c) in einem Verfahren zur Regulierung der Aktivität in Glucose- und Trigylcerid-Blutspiegeln und entzündlichen Markern bei menschlicher Adipositas.

13. Herstellung und industrielle Verwendung nach Ansprüchen 2) bis 12) des Produkts, zusammengesetzt aus einem oder mehreren Stilbenpolyphenolen und deren Derivaten zusammen ausgefällt mit lamellaren anionischen Feststoffen nach Anspruch 1), realisiert nach dem in Anspruch 2) beschriebenen Verfahren, wie es ist und/oder in Formulierungen; besonders in solchen Verfahren, bei denen eine bessere Photostabilität des Produkts Vorteile bei der Manipulation von Stilbenpolyphenolen während der Herstellung und Lagerung dieser Letzteren hervorbringt.

14. Produkt, zusammengesetzt aus Resveratrol und lamellaren anionischen Feststoffen nach Anspruch 1), realisiert nach dem in Anspruch 2) beschriebenen Verfahren zur Verwendung nach Ansprüchen 4), 5), 6), 7), 8), 9) und 10) bei der Behandlung von Pathologien, die mit der Phosphodiesterase IV-Inhibition verbunden sind.

15. Produkt, zusammengesetzt aus Resveratrol und lamellaren anionischen Feststoffen nach Anspruch 1), realisiert nach dem in Anspruch 2) beschriebenen Verfahren zur Verwendung nach Ansprüchen 4), 5), 6), 7), 8), 9) und 10) bei der Behandlung von chronisch obstruktiver Lungenerkrankung.

## Revendications

1. Produit composé d'un ou plusieurs polyphénols stilbéniques co-précipités en solides anioniques lamellaires, où:
a) les polyphénols stilbéniques
sont sélectionnés dans le groupe constitué du trans-resvératrol ; trans-3,5-diméthoxy-4'-hydroxy stilbène ; trans-3,5,4'-triméthoxy-stilbène ; trans-3,5-dihydroxy-4'-méthoxy stilbène ; trans-3,5-diacétil-4'-hydroxy-stilbène ; trans-3,5,4'-triacétil-stilbène; trans-3,5-dihydroxy-4'-acétil-stilbène ; trans-picéide; trans-pinosilvine ; cis-resvératrol ; de la raponticine ; du trans piceatannole ; de la rapontigénine ; de la delta-viniférine ; de l'epsilon-viniferina ; de l'astringine ; de la combrétastatine A-1 ; et de l'hopeaphénol ;
b) solides anioniques lamellaires signifient :
- les hydrotalcites, **caractérisées par** la formule suivante :
[M(II)₁₋ₓM(III)ₓ(OH)₂]^{x+}[Aⁿ⁻_{x/n}]^{x}_{-x/n}]^{x-} × mS
où M(II) est un métal de valence (II) préférablement choisi parmi Mg, Zn, Co, Ni, Mn, Cu ; M(III) est un métal de valence (III) préférablement choisi parmi Al, Cr, Fe, V, Co ; Aⁿ⁻ est un anion **caractérisé par** une charge négative égale à n, qui équilibre la charge positive des lamelles : m est le nombre de molécules de solvant (S), généralement de l'eau, co-intercalées, par l'unité de formule du composé;
- les bi-sels hydroxydes (HDS) **caractérisés par** la formule suivante :
[M(II)₁₋ₓM(II)₁₊ₓ(OH)_{3(1-y)}]^{x+}[Aⁿ⁺]_{(1+3y)/n} x mS
où M(II) est un métal de valence (II) préférablement choisi parmi Mg, Zn, Co, Ni, Mn, Cu ; Aⁿ⁻ est un anion **caractérisé par** une charge négative n, qui équilibre la charge positive des lamelles ; m est le nombre de molécules de solvant (S), co-intercalées, par l'unité de formule du composé.

2. Procédé de préparation d'un produit composé d'un ou plusieurs polyphénols stilbéniques tels que définis selon la revendication 1 qui co-précipitent avec des solides anioniques lamellaires tels que définis selon la revendication 1, **caractérisé par** les phases suivantes :
I) préparation d'une solution hydro-alcoolique d'un ou plusieurs polyphénols stilbéniques ;
II) préparation d'une solution hydro-alcoolique de sels de métaux bivalents ou trivalents ou d'un mélange composé de divalents et trivalents ;
III) co-précipitation d'un ou plusieurs polyphénols stilbéniques dans des solides anioniques lamellaires à un pH compris entre 8 et 11 suivant le mélange des solutions décrites aux points I) et II) et l'addition d'hydroxydes ;
IV) séparation du co-précipité ;
V) lavage du co-précipité ;
VI) évaporation des solvants.

3. Produit selon la revendication 1) ou procédé selon la revendication 2), dans lequel parmi les polyphénols stilbéniques sont compris à la fois ceux d'origine synthétique et naturelle, **caractérisé par** différents niveaux de pureté, compris entre 20 et 100 %.

4. Utilisation du produit composé des polyphénols stilbéniques et de leurs co-précipités avec des solides anioniques lamellaires tels que définis selon la revendication 1) et réalisé selon le procédé décrit selon la revendication 2) dans un dispositif médical.

5. Utilisation du produit composé des polyphénols stilbéniques et de leurs co-précipités avec des solides anioniques lamellaires tels que définis selon la revendication 1) et réalisé selon le procédé décrit selon la revendication 2) à des fins alimentaires.

6. Utilisation du produit composé des polyphénols stilbéniques et de leurs co-précipités avec des solides anioniques lamellaires tels que définis selon la revendication 1) et réalisé selon le procédé décrit selon la revendication 2) à des fins nutraceutiques.

7. Utilisation du produit composé des polyphénols stilbéniques et de leurs co-précipités avec des solides anioniques lamellaires tels que définis selon la revendication 1) et réalisé selon le procédé décrit selon la revendication 2) pour la formulation de produits pharmaceutiques.

8. Utilisation du produit composé des polyphénols stilbéniques et de leurs co-précipités avec des solides anioniques lamellaires tels que définis selon la revendication 1) et réalisé selon le procédé décrit selon la revendication 2) à des fins dermatologiques.

9. Utilisation du produit composé des polyphénols stilbéniques et de leurs co-précipités avec des solides anioniques lamellaires tels que définis selon la revendication 1) et réalisé selon le procédé décrit selon la revendication 2) à des fins cosméceutiques.

10. Utilisation selon les revendications 4), 5), 6), 7), 8) et 9), du produit composé des polyphénols stilbéniques et de leurs co-précipités avec des solides anioniques lamellaires tels que définis selon la revendication 1) et réalisé selon le procédé décrit selon la revendication 2, seul ou en association avec des ingrédients, en formulations spécifiques pour :
a) des préparations solides pour l'utilisation par voie orale et buccale : poudres, granulés, particules, capsules, pilules, comprimés, comprimés enrobés, gommes à mâcher, bolus, cachets, troches, chocolats médicamenteux ;
b) préparations liquides/semi-solides pour l'utilisation par voie orale : sirops, élixir, pâtes, jus, décoctions, suspensions, émulsions, mucilages, tisane, vins médicamenteux, apothèmes, potions ;
c) préparations d'application cutanée : crèmes, pâtes, onguents, gels, émulgels, lotions, liniments, savons médicamenteux, shampooings et shampooings médicamenteux, lapis ou crayons, cataplasmes, emplâtre, timbres, huiles, oléolites, oléose, teintures, vins, jauges médicales ;
d) préparations pour utilisations auriculaires, nasales et oro-muqueuses : lavages buccaux, gargarismes, lavages auriculaires, cône auriculaire, lavages nasaux, tampons, huiles, oléolithes, émulsions, onguents, gels, émulgels, comprimés, capsules, films, timbres, emplâtre ;
e) préparations ophtalmiques : collyre, inserts oculaires et lavages oculaires ;
f) préparations à administrer aux cavités naturelles (voies anale et vaginale) : suppositoires, capsules, comprimés, lavement, lavement entérique, lavement, irrigations, douches, mousses, tampons, bougies, ovules, mousses, gels, pâtes, émulgels, crèmes, onguents, films, suspensions, tampons ouatés, emplâtre, anneaux, tampons médicamenteux, jauges médicamenteuses ;
g) préparations à pulvériser pour utilisations topiques, par inhalation, orales, buccales.

11. Utilisation selon les revendications 4), 5), 6), 7), 8), 9) et 10), du produit composé des polyphénols stilbéniques et de leurs co-précipités avec des solides anioniques lamellaires définis selon la revendication 1) et réalisé selon le procédé décrit selon la revendication 2) dans les formulations présentées dans la revendication 10) **caractérisées par** les propriétés suivantes :
a) activités antioxydantes, anti-inflammatoires, antivirales, antidiabétiques, cardioprotectrices, neuroprotectrices, chimioprotectrices, antibiotiques, anti-ischémiques, anti-obésité, antihypertensives, anticancéreuses, antitumorales, anti-angiogéniques et antivieillissement ;
b) activités de protection contre les agents infectieux, le dysfonctionnement cardiovasculaire et la maladie néoplasique telle que le mélanome et le cancer colorectal ;
c) activité de régularisation des glycémies et triglycéridémies et de marqueurs de l'inflammation dans l'obésité humaine.

12. Co-précipité de resvératrol avec des solides anioniques lamellaires tels que définis selon la revendication 1) et réalisé selon le procédé décrit selon la revendication 2) en formulations spécifiques telles que décrites selon la revendication 10) pour l'utilisation selon les revendications 4), 5), 6), 7), 8), 9) et 10),
a) dans un procédé de traitement de l'oxidation, de l'inflammation, des infections virales, du diabète, des maladies cardiaques, des maladies neuronales, des effets défavorables des produits chimiques, des infections bactériennes, de l'ischémie, de l'obésité, de l'hypertension, du cancer, d'une tumeur, de l'angiogenèse, d'états liés au vieillissement ;
b) dans un procédé de prévention des maladies infectieuses, du dysfonctionnement cardiovasculaire et de la maladie néoplasique telle que le mélanome et le cancer colo-rectal ;
c) dans un procédé d'activité de régularisation de la glycémie et triglycéridémie et des marqueurs de l'inflammation dans l'obésité humaine.

13. Production et utilisation industrielles selon les revendications 2) à 12), du produit composé d'un ou plusieurs polyphénols stilbéniques et leurs dérivés coprécipités avec des solides anioniques lamellaires définis selon la revendication 1) réalisé selon le procédé décrit selon la revendication 2) tel quel et/ou dans des formulations ; particulièrement dans ces procédures dans lesquelles une meilleure photostabilité du produit offre des avantages en termes de manipulation des polyphénols stilbéniques durant leur production et stockage.

14. Produit composé de resvératrol et des solides anioniques lamellaires définis selon la revendication 1) réalisé selon le procédé décrit selon la revendication 2) pour l'utilisation selon les revendications 4), 5), 6), 7), 8), 9) et 10) dans le traitement de pathologies liées à l'inhibition de la phosphodiestérase IV.

15. Produit composé de resvératrol et des solides anioniques lamellaires définis selon la revendication 1) réalisé selon le procédé décrit selon la revendication 2) pour l'utilisation selon les revendications 4), 5), 6), 7), 8), 9) et 10), dans le traitement de la maladie pulmonaire obstructive chronique.
